# EUROPÄISCHE PATENTSCHRIFT

(11) **EP 4 516 271 B1**
(45) Veröffentlichungstag und Bekanntmachung des Hinweises auf die Patenterteilung: **09.12.2026**
(21) Anmeldenummer: 24195405.6
(22) Anmeldetag: 20.08.2024
(51) Int. Cl.: A61F 5/41

(54) **VORRICHTUNG ZUR VERSTEIFUNG EINER PHALLOPLASTIK EINES TRANSMANNES ODER ZUR VERSTEIFUNG EINES PENIS EINES BIOLOGISCHEN MANNES SOWIE VERFAHREN ZUR HERSTELLUNG VON ENTSPRECHENDEN VORRICHTUNGEN**
DEVICE FOR STIFFENING A PHALLOPLASTIC OF A TRANSMAN OR FOR REINFORCING A PENIS OF A BIOLOGICAL MALE AND METHOD FOR MANUFACTURING CORRESPONDING DEVICES
DISPOSITIF POUR RAIDIR UNE PROTHÈSE DE PALLOPLASTIE D'UN TRANSMAN OU POUR RAIDIR UN PÉNIS D'UN HOMME BIOLOGIQUE ET PROCÉDÉ DE FABRICATION DE DISPOSITIFS CORRESPONDANTS

(30) Priorität: 28.08.2023 DE 102023123068
(43) Veröffentlichungstag der Anmeldung: 05.03.2025
(73) Patentinhaber: Kiel, Jonathan, 53343 Wachtberg (DE)
(72) Erfinder: Kiel, Jonathan, 53343 Wachtberg (DE)
(74) Vertreter: dompatent

(56) Entgegenhaltungen:
- GB-A- 884 357
- US-A- 5 065 744
- US-A- 5 911 686
- US-A1- 2013 296 645
- US-A1- 2017 281 396
- US-B2- 11 337 846

## Beschreibung

Die vorliegende Erfindung betrifft eine Vorrichtung zur Versteifung einer Phalloplastik eines Transmannes oder eines Penis eines biologischen Mannes sowie entsprechende Verfahren zur Herstellung von vorbezeichneten Vorrichtungen.

Zur Nachbildung des männlichen Geschlechtsteils, insbesondere des Penis, wird operativ eine sogenannte Phalloplastik im Bereich des Unterleibs des Transmannes anatomisch korrekt angesetzt. Die Phalloplastik eines Transmannes wird entweder aus dem Gewebe eines freien Radialis-Lappen (Unterarmlappen) oder alternativ aus einem Anterolateral-Lappen (vorderer, seitlicher Oberschenkellappen) gebildet und an anatomisch korrekter Stelle im Bereich des Schambeins des Transmannes implantiert bzw. dort angesetzt bzw. transplantiert. Operativ wird das vorbezeichnete Gewebe zur Nachbildung eines Penis geformt, wobei dieser nachgebildete Penis, welcher als Phalloplastik bezeichnet wird, über keinen Schwellkörper verfügt. Die Vorsehung einer Glansplastik bzw. Eichelnachbildung erfolgt optional durch Transplantation eines zusätzlichen Hautstreifens an dem distalen Ende der Phalloplastik. Das transplantierte Gewebe, welches die Phallo- und/oder die Glansplastik bildet, muss jederzeit durchblutet sein, um ein Absterben des transplantierten Gewebes zu verhindern.

Aufgrund des fehlenden Schwellkörpers kann sich die Phalloplastik des Transmannes unter sexueller Erregung weder versteifen noch anschwellen und es kann folglich mit der Phalloplastik kein Geschlechtsverkehr ausgeübt werden. Aus dem Stand der Technik ist es bekannt, zur Abhilfe in die Phalloplastik entsprechende Hohlkörper zu integrieren, welche sich bei gewünschter Versteifung der Phalloplastik mit einem Fluid befüllen lassen, um somit einen künstlichen Schwellkörper nachzubilden. Das Implantat benötigt jedoch weitere kostenintensive operative Eingriffe.

Ähnliche Probleme ergeben sich bei biologischen Männern mit Erektionsstörungen, wobei der natürliche Schwellkörper nicht mehr oder lediglich unzureichend anschwillt und ebenfalls ein Geschlechtsverkehr nicht mehr ausgeübt werden kann. Bei biologischen Männern ist es bekannt, die Erektionsstörungen ebenfalls über etwaige Implantate oder den Einsatz von Medikamenten zu behandeln. Die Implantate sind jedoch wieder sehr kostenintensiv und mit operativen Eingriffen verbunden. Die Medikamente haben häufig ungewünschte Nebenwirkungen.

Aus der US 5 065 744 A und der US 5 911 686 A sind bereits Vorrichtungen zur Versteifung des männlichen Geschlechtsteils, wie insbesondere des Penis, bekannt geworden, welche jeweils die Oberbegriffsmerkmale der Ansprüche 1 und 2 offenbaren. Die offenbarten Vorrichtungen weisen den Nachteil auf, dass die offenbarten proximalen und distalen Anlageelemente nicht bzw. nur in einem unzureichenden Maße an die Anatomie des Penis bzw. der Phalloplastik angepasst sind und während der Benutzung zu Schmerzen bei den Benutzern führen können.

Ausgehend von den vorbezeichneten Nachteilen des Standes der Technik ist es die Aufgabe der vorliegenden Erfindung, den Bedürfnissen des Nutzers angepasste Vorrichtungen zur Versteifung einer Phalloplastik eines Transmannes oder des Penis eines biologischen Mannes sowie entsprechende Herstellungsverfahren zur Herstellung von entsprechenden Vorrichtungen bereitzustellen, welche die Notwendigkeit eines medizinischen Eingriffs beispielsweise zur Implantation einer Versteifungsvorrichtung gänzlich vermeiden und insbesondere auf den Einsatz von Medikamenten zu verzichten.

Gemäß einem ersten Aspekt betrifft die vorliegende Erfindung eine Vorrichtung zur Versteifung einer Phalloplastik eines Transmannes (F2M - Female-to-Male). Die erfindungsgemäße Vorrichtung umfasst mindestens einen biegesteifen Stützhalter und mindestens ein elastisches Halteelement, wobei der Stützhalter ein proximales Anlageelement, ein distales Anlageelement und mindestens eine Stützstrebe umfasst.

Die vorliegende Erfindung geht aus von dem Begriff einer Phalloplastik eines Transmannes, welche sich entlang einer Längsachse der Phalloplastik von einem proximalen Bereich als Ansatz der Phalloplastik an dem Schambein eines Transmannes zu einem gegenüberliegenden distalen freien Ende der Phalloplastik erstreckt, wobei die Phalloplastik lediglich optional im Bereich des distalen Endes eine Glansplastik aufweist.

Zur Bezugnahme soll im Rahmen der Erfindung definiert werden, dass sich eine Phalloplastik von einem proximalen Bereich bzw. proximalen Ende, an welchen der nachgebildete Penisschaft der Phalloplastik an das Körpergewebe des Transmannes bzw. genauer an das Gewebe im Bereich des Schambeines angesetzt ist, zu einem gegenüberliegenden freien distalen Ende bzw. Bereich des nachgebildeten Penisschaftes. Die Phalloplastik verläuft dabei entlang einer Längsachse des Penisschaftes von dem proximalen Ende zu dem gegenüberliegenden distalen Ende. Im Bereich des distalen Endes wird lediglich optional weiteres Körpergewebe zur Nachbildung der Eichel des Penis angesetzt, welche als Glansplastik bezeichnet wird.

Das proximale Anlageelement ist als anatomisch geformter bogenförmiger Ringabschnitt mit umfangsmäßig daran anschließenden Stützkragen zur proximalen Stützung der Phalloplastik ausgebildet. Das proximale Anlageelement ist anatomisch an den Umfang und die Form des unteren Ansatzes der Phalloplastik mit dem Schambein des Transmannes angepasst.

Das distale Anlageelement ist wiederum als anatomisch geformter bogenförmiger Ringabschnitt zur distalen Stützung der Phalloplastik ausgebildet und gemäß einer ersten Option anatomisch an den Umfang der Phalloplastik im Bereich des distalen Endes der Phalloplastik bei einer Phalloplastik ohne Glansplastik oder gemäß einer zweiten Option anatomisch an den Umfang der Phalloplastik in proximaler Richtung benachbart zu einer Glansplastikwulst der Phalloplastik ausgebildet.

Die Stützstrebe ist an die Länge der Phalloplastik bzw. genauer an die Länge des durch die Phalloplastik nachgebildeten Penisschaftes angepasst und zur Erstreckung entlang einer Längsachse der Phalloplastik bzw. des nachgebildeten Penisschaftes zwischen dem proximalen und dem distalen Anlageelement ausgebildet, wobei ein erstes proximales Ende der mindestens einen Stützstrebe mit dem proximalen Anlageelement und ein zweites gegenüberliegendes distales Ende mit dem distalen Anlageelement verbunden ist.

Das elastische Halteelement ist dazu ausgebildet in dem Bereich des distalen Anlageelementes angeordnet zu werden bzw. mit diesem trennbar verbunden zu werden und zusammen mit diesen eine geschlossene Ringstruktur zur umfangmäßigen Umschließung und distalen Stützung der Phalloplastik auszubilden.

Die vorbezeichnete Vorrichtung zur Versteifung einer Phalloplastik dient der Versteifung der Phalloplastik eines Transmannes, um die Phalloplastik für einen penetrierenden Geschlechtsverkehr zu versteifen. Das proximale Anlageelement ist anatomisch geformt als bogenförmiger Ringabschnitt, wobei sich an den Ringabschnitt umfangsmäßig ein Stützkragen anschließt, welcher ebenfalls anatomisch geformt ist an den Bereich des unteren Ansatzes der Phalloplastik mit dem Schambein des Transmannes. Das proximale Anlageelement dient dabei der proximalen Stützung der Phalloplastik, wobei sich das Anlageelement im Bereich des proximalen Übergangs der Phalloplastik zu dem Schambein anlegt, wobei insbesondere der bogenförmige Ringabschnitt zur Anlage am proximalen Ende der Phalloplastik kommt und der Stützkragen zur Anlage an dem Schambein gelangt. Das proximale Anlageelement bewirkt insbesondere durch die Abstützung gegenüber dem Schambein eine Biegeversteifung in proximalen Bereich.

Das distale Anlageelement ist als anatomisch geformter bogenförmiger Ringabschnitt zur umfangsmäßigen teilweise Umfangung und Anlage in einem proximalen Bereich der Phalloplastik angeordnet, wobei als proximaler Bereich der Bereich eines distalen Endes der Phalloplastik bei einer Phalloplastik ohne Glansplastik oder ein Bereich benachbart zu einer Glansplastikwulst einer Phalloplastik mit einer Glansplastik bezeichnet ist.

Die beanspruchte erfindungsgemäße Vorrichtung ist zur Versteifung anatomisch an die jeweilige Größe und die Form der individuellen Phalloplastik eines die erfindungsgemäß Vorrichtung nutzenden Transmannes spezifisch angepasst. Die Form als auch die Abmessungen der jeweiligen Phalloplastik eines Transmannes unterscheiden sich aufgrund der unterschiedlichen körperlichen Gegebenheiten und so ist bevorzugt eine spezifische Anpassung der erfindungsgemäßen Vorrichtung an den jeweiligen intendierten Nutzer der Vorrichtung vorzusehen.

Die erfindungsgemäße Vorrichtung ermöglicht eine Versteifung der Phalloplastik eine Transmannes ohne dabei den regulären Blutfluss in dem Gewebe des Transmannes, welche die Phalloplastik ausbildet zu unterbinden oder zu verzögern. Durch die anatomischen Anpassungen insbesondere der biegesteifen Teile der Stützstrebe erfährt der Nutzer der Vorrichtung kein unangenehmes Gefühl insbesondere werden auftretende Kräfte und/oder Biegemomente gleichmäßig durch die Vorrichtung auf die Körperanatomie des Nutzers übertragen. Durch die anatomische Anpassung und Formung der erfindungsgemäßen Vorrichtung wird beim Benutzen der Vorrichtung durch den Transmann nicht nur ein komfortables Gefühl erzeugt, sondern es wird insbesondere eine Stauchung oder Quetschung von Körpergewebe verhindert, so dass der Benutzer im Optimalfall die erfindungsgemäße Stützvorrichtung nicht spürt.

Als biegesteif soll im Rahmen der Erfindung ein Material verstanden werden, welches sich unter Aufbringung von externen Kräften nicht oder lediglich in geringem Ausmaß elastisch verformen lässt.

Gemäß einem zweiten, alternativen Aspekt betrifft die vorliegende Erfindung eine Vorrichtung zur Versteifung eines Penis eines biologischen Mannes. Derartige Vorrichtungen können beispielsweise bei Männern mit Erektionsstörungen Verwendung finden, wenn diese einen penetrierenden Geschlechtsverkehr ausüben wollen, ohne dabei beispielsweise auf Medikamente oder auf Implantate zurückgreifen zu wollen.

Die Vorrichtung zur Versteifung des Penis eines biologischen Mannes umfasst mindestens einen biegesteifen Stützhalter und mindestens zwei elastische Halteelemente. Der Stützhalter umfasst ein proximales Anlageelement, ein distales Anlageelement und mindestens eine Stützstrebe.

Das proximale Anlageelement ist als anatomisch geformter bogenförmiger Ringabschnitt zur proximalen Stützung des Penis ausgebildet, welche an den Umfang des unteren Ansatzes des Penis mit dem Hoden des Mannes bzw. an den Bereich des Übergangs von Penisschaft zu dem Hoden angepasst ist.

Das distale Anlageelement ist als anatomisch geformter bogenförmiger Ringabschnitt zur distalen Stützung ausgebildet, welcher an den Umfang des Penis im Bereich des Übergangs vom Penisschaft zur Peniseichel ausgebildet ist.

Die mindestens eine Stützstrebe ist an die Länge des Penisschafftes angepasst und zur Erstreckung entlang der Längsachse des Penisschafftes zwischen dem proximalen und dem distalen Anlageelement ausgebildet, wobei ein erstes proximales Ende der mindestens eine Stützstrebe mit dem proximalen Anlageelement und ein zweites gegenüberliegendes distales Ende mit dem distalen Anlageelement verbunden ist.

Die mindestens zwei elastischen Halteelemente sind jeweils dazu ausgebildet, in dem Bereich des distalen oder proximalen Anlageelementes angeordnet zu werden und zusammen mit dem jeweiligen Halteelement jeweils eine geschlossene Ringstruktur zur umfangsmäßigen Umschließung und Stützung des Penis auszubilden. Anders als bei der Vorrichtung zu Stützung einer Phalloplastik eines Transmannes wird die Vorrichtung zur Stützung des Penis eines biologischen Mannes zwar anatomisch angepasst ausgeführt bei Anordnung der elastischen Halteelement an dem jeweiligen Halteelement wird jedoch eine geschlossene Ringstruktur ausgebildet, welche einen spezifischen Druck auf den Penis bewirkt, wodurch bewusst der Blutfluss reduziert bzw. minimiert wird. Anders als bei einer Phalloplastik ist bei der Vorrichtung zur Stützung des Penis eines biologischen Mannes ein gewisser Blutstau gewünscht, um eben eine Versteifung des Schwellkörpers des Penis des biologischen Mannes zu erreichen.

Erfindungsgemäß kann es vorgesehen werden, dass der Stützhalter aus einem Material mit einer höheren Biegesteifigkeit als das Material des elastischen bzw. der elastischen Halteelemente ausgebildet wird. Dies hat den Vorteil, dass der Stützhalter eine biegesteife tragende bzw. stützende Grundstruktur ausbildet, welche anatomisch korrekt an die Phalloplastik bzw. den Penis des Nutzers ausgebildet ist, welche über die elastischen Halteelemente, welche eine geringere Biegesteifigkeit aufweisen eben elastisch gegenüber der Phalloplastik bzw. dem Penis festgelegt werden kann. Die erfindungsgemäße Vorrichtung stellt somit durch Ihre biegesteifen Teile einen erigierten bzw. versteiften Penis nach und ermöglicht dadurch dem Transmann oder dem biologischen Mann mit erektiler Störung bzw. Dysfunktion Geschlechtsverkehr auszuüben.

Es kann vorgesehen werden, dass der Stützhalter bevorzugt aus einem Hautkontakt geeigneten Kunststoffmaterial ausgebildet ist und insbesondere zumindest ein Material ausgewählt aus der Gruppe von PETG, Polyamid, PEEK und Polypropylen umfasst.

Weiterhin kann es vorgesehen werden, dass das elastische oder die elastischen Halteelemente zumindest ein Material ausgewählt aus der Gruppe von Silikon und Polyurethan umfassen.

Das proximale Anlageelement kann als nach oben offener bogenförmiger Ringabschnitt ausgebildet werden, mit daran anschließenden Stützkragen, welche zur Unterfangung des proximalen Ansatzbereiches der Phalloplastik bzw. des Penis und zur Ausbildung eines Aufnahmeraums für den proximalen Bereich der Phalloplastik bzw. des Penis eines Transmannes bzw. eines Mannes ausgebildet sind. Der vorgesehene Stützkragen dient dabei der Abstützung der Vorrichtung im Bereich des Schambeins bzw. des Hodens des Mannes, wodurch eine zusätzliche Versteifung der Phalloplastik bzw. des zu stützenden Penis erreicht werden kann, nämlich dahingehend, dass durch den zusätzlichen Stützkragen eine biegesteife Struktur im Bereich des proximalen Ansatzes angelagert wird, wodurch ein Abknicken der Phalloplastik bzw. des Penis im Bereich des proximalen Endes insbesondere beim penetrierendem Geschlechtsverkehr verhindert werden kann.

Das distale Anlageelement kann als nach unten offener bogenförmiger Ringabschnitt ausgebildet werden, welcher zur Überfangung des distalen Bereichs der Phalloplastik eines Transmannes bzw. des Penis eines biologischen Mannes und zur Ausbildung eines nach unten offenen Aufnahmeraums für den Penis bzw. die Phalloplastik vorgesehen ist.

Der Stützhalter kann in einer bevorzugten Ausführungsform mindestens zwei Stützstreben umfassen, welche sich jeweils von einem distalen oberen Ende des proximalen Anlageelements zu einem gegenüberliegenden proximalen unteren Ende des distalen Anlageelementes erstrecken, wobei die mindestens zwei Stützstreben bevorzugt entlang gegenüberliegender Seiten der Phalloplastik bzw. des Penis verlaufend ausgebildet sind.

Besonders bevorzugt kann es vorgesehen werden, dass die beiden gegenüberliegend ausgebildeten Stützstreben orthogonal zu der Richtung der Längsachse der Phalloplastik bzw. des Penis einen Abstand aufweisen, welcher an den Umfang der Phalloplastik bzw. des Penis angepasst ist.

Insbesondere zur vereinfachten Reinigung kann es vorgesehen werden, dass die beiden Anlageelemente und die mindestens eine Stützstrebe einstückig aus einem Material und/oder einstückig ausgebildet werden. Dies hat den weiteren Vorteil, dass der Stützhalter nahtlos bzw. ohne Übergänge oder Sprünge ausgebildet wird, was weiterhin der Vermeidung von möglichen unangenehmen Druckstellen für den Nutzer dient.

Erfindungsgemäß kann es vorgesehen werden, dass der Stützhalter zumindest eine Aussparung aufweist, bei der bevorzugt in die zumindest eine Aussparung ein Funktionselement einsetzbar ist.

Die Vorsehung von mindestens einer Aussparung weist den Vorteil auf, dass die Stützvorrichtung aufgrund der Materialausnehmung weniger Material aufweist und damit mit einem geringeren Gewicht ausgestattet werden kann, wobei gleichzeitig weiterhin die Atmung des in der Stützvorrichtung aufgenommenen Körpergewebes sichergestellt werden kann. Besonders vorteilhaft werden Aussparungen in der Struktur des Stützhalters ausgebildet, welche auch während der Nutzung keine Lasten bzw. lokal geringe Lasten erfahren. Insbesondere kann die Struktur des Stützträgers einer Topologieoptimierung unterzogen werden.

Weiterhin können in der mindestens einen Aussparung Funktionselemente wie beispielsweise ein Druck- oder ein Vibrationselement aufgenommen werden. Die Funktionselemente können den Komfort des Nutzers der Vorrichtung bzw. insbesondere das Lustempfinden während der Nutzung steigern.

Es kann vorgesehen werden, dass an dem distalen Anlageelement oder an dem proximalen Anlageelement mindestens eine Aussparung oder mindestens einen Vorsprung zur Befestigung des elastischen Halteelements an dem jeweiligen Anlageelement ausgebildet ist. Die Vorsehung einer Aussparung oder eines Vorsprungs weist den Vorteil auf, dass sich das elastische Halteelement über den vorbezeichneten Vorsprung oder die Aussparung mit dem jeweiligen Anlageelement zur Ausbildung einer den Penis bzw. die Phalloplastik umschließenden Ringstruktur zusammenfügen lässt. Auch lässt sich die gebildete Ringstruktur durch den Nutzer auch leicht lösen.

Es kann vorgesehen werden, dass der Stützhalter durch ein dreidimensionalen Druckverfahren oder mittels eines Spritzgussverfahren hergestellt wird. Das dreidimensionale Druckverfahren ermöglicht auf besonders einfache und kostengünstige Art und Weise einen an den Nutzer spezifisch angepassten Stützhalter herzustellen.

Weiterhin kann es vorgesehen werden, dass der Stützhalter als auch das mindestens eine elastische Element mittels eines dreidimensionalen Druckverfahrens hergestellt werden, wobei das elastische Element mittels dem dreidimensionalen Druckverfahren an den Stützhalter angedruckt wird um eine einstückige Ausführungsform zu erhalten.

Gemäß einem weiteren Aspekt betrifft die vorliegende Erfindung ein Verfahren zu Herstellung einer Vorrichtung zur Stützung einer Phalloplastik eines Transmannes umfassend die Verfahrensschritte:
a) Messen des Umfanges und der Form des unteren Ansatzes einer Phalloplastik mit dem Schambein eines Transmannes;
b) Messen des Umfangs der Phalloplastik des Transmannes ohne Glansplastik im Bereich des distalen Endes der Phalloplastik oder Messen des Umfangs der Phalloplastik des Transmannes benachbart zu der Glansplastikwulst;
c) Messen des Umfangs der Phalloplastik in der Mitte der Phalloplastik zwischen proximalem Ansatz und dem distalen Ende der Phalloplastik;
d) Messen der Länge der Phalloplastik vom Ansatz bis zur Spitze der Phalloplastik
e) Fertigen des Stützhalters und bevorzugt des elastischen Halteelementes für die Phalloplastik anhand der Messwerte aus den Schritten a) bis d).

Gemäß einem alternativen weiteren Aspekt betrifft die vorliegende Erfindung ein Verfahren zur Herstellung einer Vorrichtung zur Stützung des Penis eines biologischen Mannes umfassend die Verfahrensschritte:
a) Messen des Umfanges und der Form des unteren proximalen Ansatzes eines Penis mit dem Schambein eines biologischen Mannes;
b) Messen des Umfangs des Penis im Bereich des Überganges des Penisschaftes zur Peniseichel;
c) Messen des Umfangs des Penisschaftes in der Mitte der zwischen dem proximalen Ansatz und dem Übergang zur Peniseichel;
d) Messen der Länge des Penisschaftes vom proximalen Ansatz bis zum Übergang zur Peniseichel;
e) Fertigen des Stützhalters und bevorzugt der elastischen Halteelemente für den Penis anhand der Messwerte aus den Schritten a) bis d).

In den beiden vorbezeichneten Verfahren kann es vorzugsweise vorgesehen werden, dass nach dem Schritt d) zusätzlich ein Modell, insbesondere eine virtuelles Geometriemodell des Stützhalters erstellt wird, wobei die Dimensionierung der beiden Anlageelemente sowie der mindestens eines Stützstrebe und bevorzugt des elastischen Halteelementes aufgrund der Messwerte aus den vorangegangenen Verfahrensschritten erfolgt und wobei im Schritt e) ein Stützhalter anatomisch korrekt und angepasst anhand des Models gefertigt wird.

Im Nachfolgenden werden unter Bezugnahme auf die beigefügten Figuren beispielhafte erfindungsgemäße Ausführungsformen der vorliegenden Erfindung erläutert.

Es zeigen:
Figur 1 eine schematische seitliche Ansicht einer Phalloplastik umfassend eine Glansplastik eines Transmannes; die
Figuren 2a bis 2d eine erste beispielhafte Ausführungsform einer erfindungsgemäßen Vorrichtung zur Versteifung einer Phalloplastik eines Transmannes, sowie die
Figuren 3a und 3b eine zweite beispielhafte Ausführungsform einer erfindungsgemäßen Vorrichtung zur Versteifung des Penis eines biologischen Mannes.

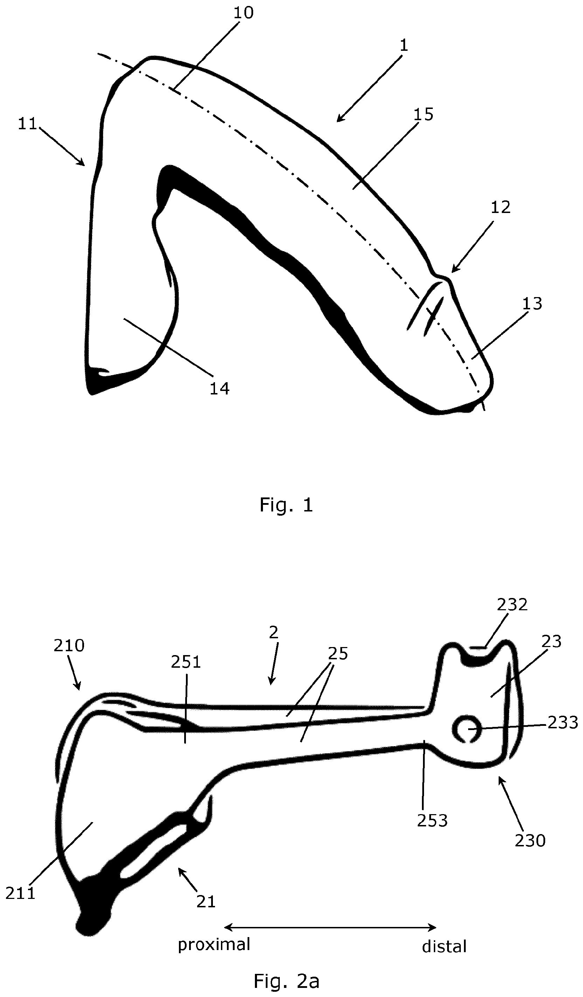

Die Figur 1 zeigt zunächst zur Verdeutlichung der Anatomie, auf welche vorliegend Bezug genommen werden soll, eine Phalloplastik 1 eines Transmannes. Die in der Figur 1 dargestellte Phalloplastik 1 umfasst eine Glansplastik 13 sowie eine Hodenplastik 14. Grundsätzlich erstreckt sich die Phalloplastik von dem Schambein 11 des Transmannes entlang eines nachgebildeten Penisschaftes von dem proximalen Ende im Bereich des Schambeins 11 zu dem gegenüberliegenden distalen Ende des Schaftes 15 wobei wie in Figur 1 dargestellt im Bereich des distalen Endes der Phalloplastik 1 optional eine Glansplastik 13 sowie eine Glansplastikwulst 12 im Bereich des Übergangs zwischen dem Schaft 15 der Phalloplastik 1 und der Glansplastik 13 ausgebildet wird, zur Nachbildung des Penis sowie optional des Hodens.

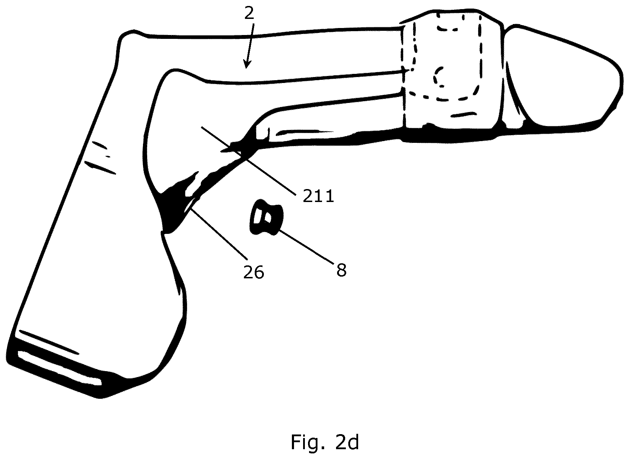

In den Figuren 2a bis 2d ist eine erste beispielhafte Ausführungsform einer erfindungsgemäßen Vorrichtung zur Versteifung einer Phalloplastik 1 eines Transmannes dargestellt, wobei die Figur 2a lediglich die Anteile des Stützhalters 2 der Vorrichtung zur Versteifung darstellt. Die dargestellte beispielhafte Ausführungsform des erfindungsgemäßen Stützhalters 2 umfasst zunächst ein proximales Anlageelement 21 welches in der dargestellten Ausführungsform über zwei Stützstreben 25 mit einem distalen Anlageelement 23 verbunden ist. Das proximale Anlageelement 21 ist als anatomisch geformter bogenförmiger Ringabschnitt mit umfangsmäßig daran anschließenden Stützkragen 211 zur proximalen Stützung der Phalloplastik ausgebildet und an den Umfang und die Form des unteren Ansatzes der Phalloplastik 1 mit dem Schambein 11 des Transmannes angepasst.

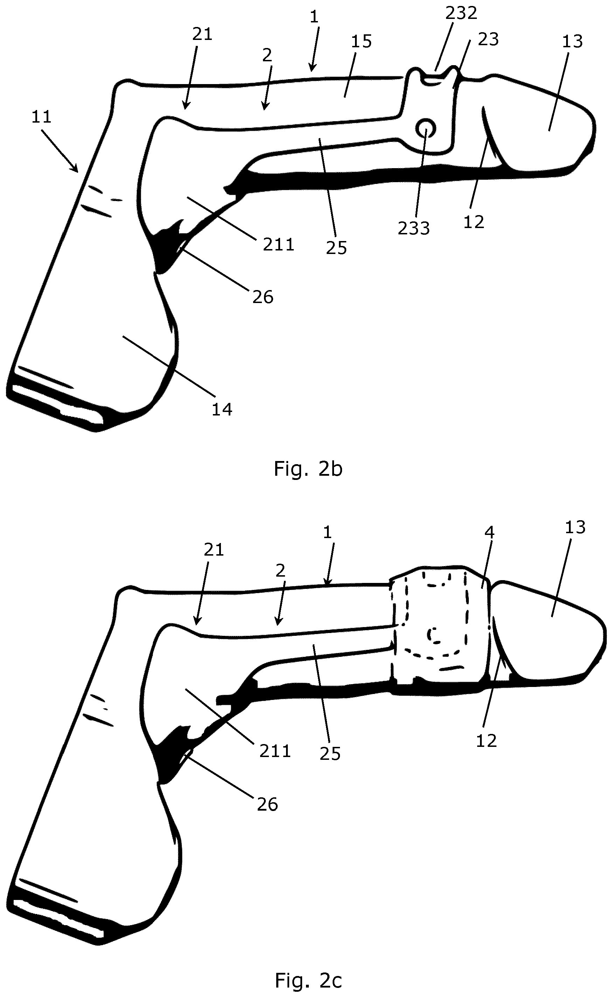

Die Figur 2b zeigt die Ansicht einer erfindungsgemäßen Stützstrebe 2 welche zur Versteifung einer Phalloplastik 1 daran angeordnet ist. Zur Verdeutlichung des Verlaufs und der Merkmale ist der Stützhalter 2 in der Figur 2b noch ohne elastisches Element 4 dargestellt. Wie aus der Figur 2b entnehmbar ist, ist das distale Anlageelement 23 als anatomisch geformter bogenförmiger Ringabschnitt zur distalen Stützung der Phalloplastik 1 ausgebildet und an den Umfang der Phalloplastik 1 im Bereich des distalen Endes der Phalloplastik 1 bei einer Phalloplastik ohne Glansplastik oder wie in Figur 2b dargestellt den Umfang der Phalloplastik benachbart zu einer Glansplastikwulst 12 ausgebildet. Die beiden ausgebildeten Stützstreben 25 sind jeweils an die Länge der Phalloplastik 1 angepasst und zur Erstreckung entlang der Längsachse 10 der Phalloplastik 1 von dem proximalen zu dem distalen Anlageelement 21, 23 ausgebildet.

Wie insbesondere der Figur 2a aber auch der 2b entnehmbar, ist das erste proximale Ende 251 der Stützstrebe 25 mit dem proximalen Anlagenelement 21 und ein zweites Ende 253 mit dem distalen Anlageelement verbunden. Wie der Figur 2b entnehmbar ist, ist das elastische Halteelement 4 dazu ausgebildet in dem Bereich des distalen Anlageelements 23 angeordnet zu werden und zusammen mit diesen eine geschlossene Ringstruktur zur umfangsmäßigen Umschließung der Phalloplastik und zur distalen Stützung der Phalloplastik auszubilden.

Wie über Figur 2c ersichtlich, schmiegt sich durch die anatomische Formung der Einzelelemente des erfindungsgemäßen Stützhalters 2 die Struktur des Stützhalters 2 an die Form der Phalloplastik 1 an, wobei der Schaft 15 der Phalloplastik 1 aufgrund der Stützung durch den Stützhalter 2 derart ausgerichtet ist, dass die Längsachse 10 annähernd als Gerade verläuft. Durch die Anordnung des mindestens einen elastischen Halteelements 4 wird dabei der erfindungsgemäße Stützhalter 2 an die Phalloplastik 1 angebunden bzw. gegenüber der Phalloplastik 1 festgelegt, ohne dabei die Blutzirkulation innerhalb der Phalloplastik 1 zu unterbinden. Allein aufgrund des Anliegens des Stützhalters 2 und der anatomischen Formung wird eine Stützung der Phalloplastik 1 ermöglicht, derart das die Längsachse des Schaftes 15 der Phalloplastik 1 geradlinig verläuft und eben derart gestützt bzw. versteift ist, so dass mit der Phalloplastik 1 ein Geschlechtsverkehr ausgeübt werden kann.

Funktional kann der Stützhalter 2 noch mit einer Aussparung 26 zur Anordnung eines Funktionselementes 8, beispielsweise eines Vibrationselementes zur Steigerung des Wohlbefindens ausgestattet werden.

Wie dies den Figuren 2b bis 2d entnehmbar ist, kann die erfindungsgemäße Stützstrebe 25 einstückig aus einem Material zusammen mit den beiden Anlageelementen 21, 23 gebildet werden, was den besonderen Vorteil hat, dass die derart ausgebildete Vorrichtung aufgrund der Einstückigkeit und der entsprechenden einstückigen Oberfläche vereinfacht gereinigt bzw. desinfiziert werden kann.

Das distale Anlageelement 23 kann mit mindestens einer Aussparung 232 oder mit mindestens einem Vorsprung 233 zur Befestigung des elastischen Halteelementes 4 an dem distalen Anlageelement ausgebildet werden. Der Stützhalter 2 sowie bevorzugt das mindestens eine elastische Element können über ein dreidimensionales Druckverfahren hergestellt werden.

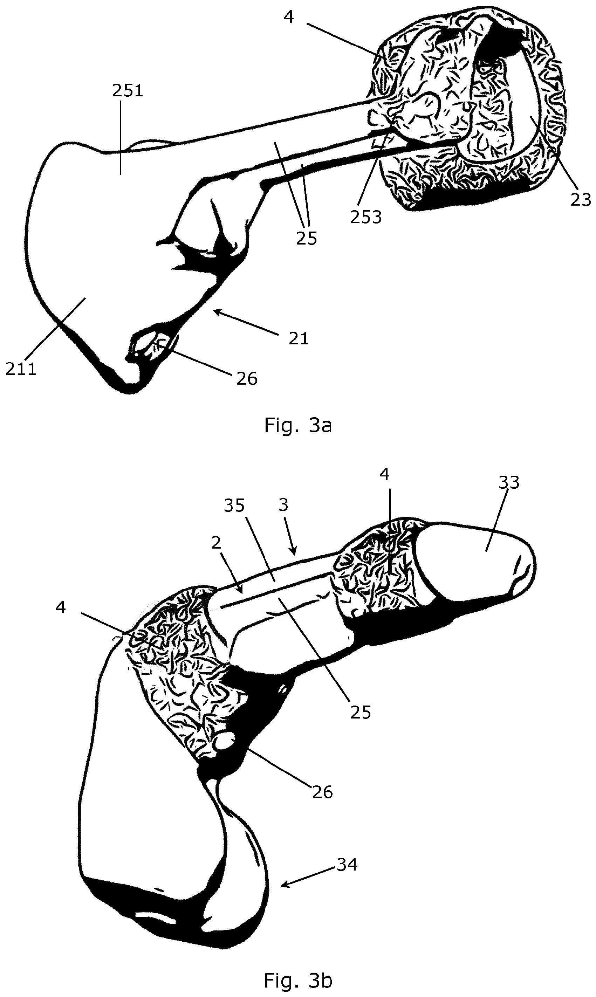

In den Figuren 3a und 3b ist eine alternative erfindungsgemäße Vorrichtung zur Versteifung des Penis 3 eines biologischen Mannes gestellt. Im Unterschied zu der Phalloplastik eines Transmannes, wobei die erfindungsgemäße Vorrichtung die Phalloplastik 1 zwar für penetrierenden Geschlechtsverkehr stützt, wobei jedoch keine Blutstauung der Phalloplastik 1 des Transmannes erfolgen darf, ist im Gegensatz bei Vorrichtung zur Versteifung des Penis 3 eines biologischen Mannes gerade wünschenswert durch die mindestens zwei elastischen Halteelemente eine Blutstauung in dem Penis 3 und insbesondere im Bereich des Penisschaftes 35 des Penis 3 des biologischen Mannes herbeizuführen um eben eine Blutstauung im Bereich des Schwellkörpers des Penis 3 des biologischen Mannes und damit einhergehend eine Versteifung des Penis 3 des biologischen Mannes hervorzurufen.

Die Vorrichtung zur Versteifung des Penis 3 des biologischen Mannes welche in den Figuren 3a und 3b dargestellt ist, umfasst wiederum mindestens einen biegesteifen Stützhalter jedoch mindestens zwei elastische Halteelemente 4. Der Stützhalter umfasst wiederum ein proximales Anlageelemente 21 sowie ein distales Anlageelement 23 und der dargestellten Ausführungsform zwei gegenüberliegende Stützstreben 25. Das proximale Anlageelement 21 ist als anatomisch geformter bogenförmiger Ringabschnitt zur proximalen Stützung des Penis 3 ausgebildet, welcher an den Umfang des unteren Ansatzes des Penis 3, genauer des Penisschaftes 35 mit dem Hoden 34 des biologischen Mannes angepasst ist. Das distale Anlageelement 23 ist als anatomisch geformter bogenförmiger Ringabschnitt zur distalen Stützung des Penis 3 bzw. des Penisschaftes 35 ausgebildet, welcher an den Umfang des Penis 3 im Bereich des Übergangs des Penisschaftes 35 zur Peniseichel 33 ausgebildet ist. Die Stützstrebe 25 ist dabei an die Länge des Penisschaftes 35 angepasst und zur Erstreckung entlang der Längsachse des Penisschaftes 35 angepasst. Ein erstes proximales Ende 251 der Stützstreben 25 ist mit dem proximalen Anlageelement 21 und ein zweites gegenüberliegendes distales Ende 253 mit dem distalen Anlageelement 23 verbunden.

Die mindestens zwei elastischen Halteelemente 4 sind jeweils dazu ausgebildet in dem Bereich des distalen und proximalen Anlageelementes 21, 23 angeordnet zu werden und zusammen mit diesen jeweils eine geschlossene Ringstruktur zur umfangsmäßigen Umschließung und Stützung des Penis auszubilden und wie bereits zuvor erläutert eine Blutstauung im Bereich des Penis 3 des biologischen Mannes in den entsprechenden Anordnungsbereichen zu bewirken.

## Patentansprüche

1. Vorrichtung zur Versteifung einer Phalloplastik (1) eines Transmannes, F2M, umfassend:
mindestens einen biegesteifen Stützhalter (2) und mindestens ein elastisches Halteelement (4),
wobei der Stützhalter (2) umfasst:
- ein proximales Anlageelement (21),
- ein distales Anlageelement (23), und
- mindestens eine Stützstrebe (25),
wobei das distale Anlageelement (23) als anatomisch geformter bogenförmiger Ringabschnitt zur distalen Stützung der Phalloplastik (1) ausgebildet ist und
- an den Umfang der Phalloplastik (1) im Bereich des distalen Endes der Phalloplastik (1) bei einer Phalloplastik (1) ohne Glansplastik
oder
- an den Umfang der Phalloplastik (1) benachbart zu einer Glansplastikwulst (12) der Phalloplastik (1) ausgebildet ist;
wobei die Stützstrebe (25) an die Länge der Phalloplastik (1) angepasst ist und zur Erstreckung entlang der Längslachse (10) der Phalloplastik (1) zwischen dem proximalen und dem distalen Anlageelement (21, 23) ausgebildet ist; und wobei ein erstes proximales Ende (251) der mindestens einen Stützstrebe (25) mit dem proximalen Anlageelement (21) und ein zweites distales Ende (253) mit dem distalen Anlageelement (23) verbunden ist;
**dadurch gekennzeichnet, dass**
das proximale Anlageelement (21) als anatomisch geformter bogenförmiger Ringabschnitt mit umfangsmäßig daran anschließenden Stützkragen (211) zur proximalen Stützung der Phalloplastik (1) ausgebildet ist und an den Umfang und die Form des unteren Ansatzes der Phalloplastik (1) mit dem Schambein (11) des Transmannes angepasst ist; und
dass das elastische Haltelement (4) ausgebildet ist in dem Bereich des distalen Anlageelementes (23) angeordnet zu werden und zusammen mit diesem eine geschlossene Ringstruktur zur umfangmäßigen Umschließung und distalen Stützung der Phalloplastik (1) auszubilden.

2. Vorrichtung zur Versteifung des Penis (3) eines biologischen Mannes, umfassend:
mindestens einen biegesteifen Stützhalter (2) und mindestens zwei elastische Halteelemente (4),
wobei der Stützhalter (2) umfasst:
- ein proximales Anlageelement (21),
- ein distales Anlageelement (23), und
- mindestens eine Stützstrebe (25),
wobei das distale Anlageelement (23) als anatomisch geformter bogenförmiger Ringabschnitt zur distalen Stützung ausgebildet ist, welcher an den Umfang des Penis (3) im Bereich des Übergangs zur Peniseichel (33) ausgebildet ist;
wobei die Stützstrebe (25) an die Länge des Penisschaftes (35) angepasst ist und zur Erstreckung entlang der Längslachse des Penisschaftes (35) zwischen dem proximalen und dem distalen Anlageelement (21, 23) ausgebildet ist,
wobei ein erstes proximales Ende (251) der mindestens einen Stützstrebe (25) mit dem proximalen Anlageelement (21) und ein zweites distales Ende (253) mit dem distalen Anlageelement (23) verbunden ist; und
**dadurch gekennzeichnet, dass**
das proximale Anlageelement (21) als anatomisch geformter bogenförmiger Ringabschnitt zur proximalen Stützung des Penis (3) ausgebildet ist, welcher an den Umfang des unteren Ansatzes des Penis (3) mit dem Hoden (34) des Mannes angepasst ist; unddass die mindestens zwei elastischen Haltelemente (4) jeweils dazu ausgebildet sind in dem Bereich des distalen und proximalen Anlageelementes (21, 23) angeordnet zu werden und zusammen mit diesem jeweils eine geschlossene Ringstruktur zur umfangmäßigen Umschließung und Stützung des Penis (3) auszubilden.

3. Vorrichtung nach Anspruch 1 oder 2, wobei der Stützhalter (2) aus einem Material mit einer höheren Biegesteifigkeit als das Material des elastischen Halteelementes gebildet ist.

4. Vorrichtung nach einem der Ansprüche 1 bis 3, wobei der Stützhalter (2) aus einem hautkontaktgeeignetem Kunststoffmaterial ausgebildet ist und insbesondere zumindest ein Material ausgewählt aus der Gruppe von: PETG, Polyamid, PEEK und Polypropylen umfasst.

5. Vorrichtung nach einem der vorausgehenden Ansprüche, wobei das elastische Haltelement (4) zumindest ein Material ausgewählt aus der Gruppe von Silikon und Polyurethan umfasst.

6. Vorrichtung nach einem der vorausgehenden Ansprüche, wobei das proximale Anlageelement (21) als nach oben offener bogenförmiger Ringabschnitt mit daran anschließendem Stützkragen ausgebildet ist zur Unterfangung des proximalen Ansatzbereiches der Phalloplastik (1) und zur Ausbildung eines Aufnahmeraums (210) für den proximalen Bereich der Phalloplastik (1) eines Transmannes.

7. Vorrichtung nach einem der vorausgehenden Ansprüche, wobei das distale Anlageelement (23) als nach unten offener bogenförmiger Ringabschnitt ausgebildet ist zur Überfangung des distalen Bereiches der Phalloplastik (1) eines Transmannes und zur Ausbildung eines nach unten offenen Aufnahmeraums (230).

8. Vorrichtung nach einem der vorausgehenden Ansprüche, wobei der Stützhalter (2) mindestens zwei Stützstreben (25) umfasst, welche sich jeweils von einem distalen oberen Ende des proximalen Anlageelements (21) zu einem proximalen unteren Ende des distalen Anlageelementes (23) erstrecken, wobei die mindestens zwei Stützstreben (25) bevorzugt entlang gegenüberliegender Seiten der Phalloplastik (1) verlaufend ausgebildet sind.

9. Vorrichtung nach einem der vorausgehenden Ansprüche, wobei die beiden Anlageelemente (21, 23) und die mindestens eine Stützstrebe (2) einstückig aus einem Material gebildet sind.

10. Vorrichtung nach einem der vorausgehenden Ansprüche, wobei an dem distalen Anlageelement (23) mindestens eine Aussparung (232) und/oder mindestens ein Vorsprung (233) zur Befestigung des elastischen Haltelementes (4) an dem distalen Anlageelement (23) ausgebildet ist.

11. Vorrichtung nach einem der vorausgehenden Ansprüche, wobei der Stützhalter (2) mittels eines dreidimensionalen Druckverfahrens oder mittels eines Spritzgussverfahrens hergestellt ist.

12. Vorrichtung nach einem der vorausgehenden Ansprüche, wobei der Stützhalter (2) mittels eines dreidimensionalen Druckverfahrens hergestellt ist und wobei das mindestens eine elastische Element (4) ebenfalls mittels eines dreidimensionalen Druckverfahren hergestellt und an dem Stützhalter (2) angedruckt ist.

13. Verfahren zur Herstellung einer Vorrichtung nach einem der Ansprüche 1 oder 3 bis 12 umfassend die Schritte:
a) Messen des Umfanges und der Form des unteren Ansatzes einer Phalloplastik (1) mit dem Schambein (11) eines Transmannes;
b) Messen des Umfangs der Phalloplastik (1) des Transmannes ohne Glansplastik (13) im Bereich des distalen Endes der Phalloplastik (1) oder Messen des Umfangs der Phalloplastik (1) des Transmannes mit Glansplastik (13) benachbart zu der Glansplastikwulst (12);
c) Messen des Umfangs der Phalloplastik (1) in der Mitte der Phalloplastik (19 zwischen proximalem Ansatz und dem distalen Ende der Phalloplastik (1);
d) Messen der Länge der Phalloplastik (1) vom Ansatz bis zur Spitze der Phalloplastik (1);
e) Fertigen des Stützhalters (2) und bevorzugt des elastischen Halteelementes (4) für die Phalloplastik (2) anhand der Messwerte aus den Schritten a) bis d).

14. Verfahren zur Herstellung einer Vorrichtung nach einem der Ansprüche 2 bis 12 umfassend die Schritte:
a) Messen des Umfanges und der Form des unteren proximalen Ansatzes eines Penis mit dem Schambein eines biologischen Mannes;
b) Messen des Umfangs des Penis im Bereich des Überganges des Penischaftes zur Peniseichel;
c) Messen des Umfangs des Penisschaftes in der Mitte der zwischen dem proximalen Ansatz und dem Übergang zur Peniseichel;
d) Messen der Länge des Penisschaftes vom proximalen Ansatz bis zum Übergang zur Peniseichel;
e) Fertigen des Stützhalters und bevorzugt der elastischen Halteelemente für den Penis anhand der Messwerte aus den Schritten a) bis d).

15. Verfahren nach Anspruch 13 oder 14, wobei das Verfahren nach dem Schritt d) zusätzlich den Verfahrensschritt umfasst: e) Erstellen eines Modells des Stützhalters, wobei die Dimensionierung der beiden Anlageelemente, der mindestens einen Stützstrebe und bevorzugt des elastischen Halteelementes aufgrund der Messwerte aus den Verfahrensschritten a) bis d) erfolgt; und wobei im Schritt e) der Stützhalter und bevorzugt das elastische Halteelement anhand des Modells gefertigt wird.

## Claims

1. A device for stiffening a phalloplasty (1) of a trans man, F2M, comprising:
at least one flexurally rigid support bracket (2) and at least one elastic retaining element (4),
wherein the support bracket (2) comprises:
- a proximal abutment element (21),
- a distal abutment element (23), and
- at least one support strut (25),
wherein the distal abutment element (23) is configured as an anatomically shaped, arched ring section for providing distal support to the phalloplasty (1), and
- is shaped to fit the circumference of the phalloplasty (1) in the area of the distal end of the phalloplasty (1) in the case of a phalloplasty (1) without glansplasty,
or
- is shaped to fit the circumference of the phalloplasty (1) adjacent to a glansplasty ridge (12) of the phalloplasty (1);
wherein the support strut (25) is adapted to the length of the phalloplasty (1) and is configured to extend along the longitudinal axis (10) of the phalloplasty (1) between the proximal and distal abutment elements (21, 23); and wherein a first proximal end (251) of the at least one support strut (25) is connected to the proximal abutment element (21) and a second distal end (253) is connected to the distal abutment element (23);
**characterized in that**
the proximal abutment element (21) is configured as an anatomically shaped, arched ring section with a circumferentially adjoining support collar (211) for proximal support of the phalloplasty (1) and is adapted to the circumference and shape of the lower base of the phalloplasty (1) with the pubic bone (11) of the trans man; and
the elastic retaining element (4) is configured to be arranged in the area of the distal abutment element (23) and to form, together with it, a closed ring structure for circumferentially enclosing and distally supporting the phalloplasty (1).

2. The device for stiffening the penis (3) of a biological male, comprising:
at least one flexurally rigid support bracket (2) and at least two elastic retaining elements (4),
wherein the support bracket (2) comprises:
- a proximal abutment element (21),
- a distal abutment element (23), and
- at least one support strut (25),
wherein the distal abutment element (23) is configured as an anatomically shaped, arched ring section for distal support, which is shaped to fit the circumference of the penis (3) in the area of the transition to the glans penis (33);
wherein the support strut (25) is adapted to the length of the penile shaft (35) and is configured to extend along the longitudinal axis of the penile shaft (35) between the proximal and distal abutment elements (21, 23);
wherein a first proximal end (251) of the at least one support strut (25) is connected to the proximal abutment element (21) and a second distal end (253) is connected to the distal abutment element (23); and
**characterized in that**
the proximal abutment element (21) is configured as an anatomically shaped, arched ring section for providing proximal support to the penis (3), which is adapted to the circumference of the lower base of the penis (3) with the male's testicle (34); and that the at least two elastic retaining elements (4) are each configured to be arranged in the area of the distal and proximal abutment elements (21, 23) and to form, together with them, a closed ring structure for circumferentially enclosing and supporting the penis (3).

3. The device according to claim 1 or 2, wherein the support bracket (2) is made of a material having a higher flexural rigidity than the material of the elastic retaining element.

4. The device according to any one of claims 1 to 3, wherein the support bracket (2) is made of a plastic material suitable for skin contact and, in particular, at least one material selected from the group consisting of: PETG, polyamide, PEEK, and polypropylene.

5. The device according to any one of the preceding claims, wherein the elastic retaining element (4) comprises at least one material selected from the group consisting of silicone and polyurethane.

6. The device according to any one of the preceding claims, wherein the proximal abutment element (21) is configured as an upwardly open, arched ring section with an adjoining support collar for underpinning the proximal attachment region of the phalloplasty (1) and forming an accommodation space (210) for the proximal region of the phalloplasty (1) of a trans man.

7. The device according to any one of the preceding claims, wherein the distal abutment element (23) is configured as a downwardly open, arched ring section for engaging the distal region of the phalloplasty (1) of a trans man and forming a downwardly open accommodation space (230).

8. The device according to any one of the preceding claims, wherein the support bracket (2) comprises at least two support struts (25), each of which extends from a distal upper end of the proximal abutment element (21) to a proximal lower end of the distal abutment element (23), wherein the at least two support struts (25) are preferably configured to extend along opposite sides of the phalloplasty (1).

9. The device according to any one of the preceding claims, wherein the two abutment elements (21, 23) and the at least one support strut (2) are formed integrally from a single material.

10. The device according to any one of the preceding claims, wherein at least one recess (232) and/or at least one protrusion (233) is formed on the distal abutment element (23) for securing the elastic retaining element (4) to the distal abutment element (23).

11. The device according to any one of the preceding claims, wherein the support bracket (2) is manufactured using a three-dimensional printing process or an injection molding process.

12. The device according to any one of the preceding claims, wherein the support bracket (2) is manufactured using a three-dimensional printing process, and wherein the at least one elastic element (4) is also manufactured using a three-dimensional printing process and is printed onto the support bracket (2).

13. A method for manufacturing a device according to any one of claims 1 or 3 to 12, comprising the steps of:
a) measuring the circumference and shape of the lower base of a phalloplasty (1) with the pubic bone (11) of a trans man;
b) measuring the circumference of the phalloplasty (1) of a trans man without glansplasty (13) in the area of the distal end of the phalloplasty (1) or measuring the circumference of the phalloplasty (1) of the trans man with glansplasty (13) adjacent to the glansplasty ridge (12);
c) measuring the circumference of the phalloplasty (1) at the center of the phalloplasty (1) between the proximal base and the distal end of the phalloplasty (1);
d) measuring the length of the phalloplasty (1) from the base to the tip of the phalloplasty (1);
e) manufacturing the support bracket (2) and, preferably, the elastic retaining element (4) for the phalloplasty (2) based on the measured values obtained in steps a) to d).

14. A method for manufacturing a device according to any one of claims 2 to 12, comprising the steps of:
a) measuring the circumference and shape of the lower proximal base of a penis with the pubic bone of a biological male;
b) measuring the circumference of the penis at the junction between the penile shaft and the glans penis;
c) measuring the circumference of the penile shaft at the center between the proximal base and the junction with the glans penis;
d) measuring the length of the penile shaft from the proximal base to the junction with the glans penis;
e) manufacturing the support bracket and, preferably, the elastic retaining element for the penis based on the measured values obtained in steps a) to d).

15. The method according to claim 13 or 14, wherein, following step d), the method additionally comprises the method step: e) creating a model of the support bracket, wherein the dimensioning of the two abutment elements, the at least one support strut, and preferably the elastic retaining element is based on the measured values from method steps a) to d); and wherein, in step e), the support bracket and, preferably, the elastic retaining element are manufactured based on the model.

## Revendications

1. Dispositif pour rigidifier une phalloplastie (1) d'un homme trans, F2M, comprenant:
au moins un support de soutien rigide (2) et au moins un élément de maintien élastique (4),
où le support de soutien (2) comprend:
- un élément d'appui proximal (21),
- un élément d'appui distal (23), et
- au moins une entretoise de soutien (25),
où l'élément d'appui distal (23) est formé comme une section annulaire en forme d'arc de forme anatomique pour le soutien distal de la phalloplastie (1) et est formé
- au niveau de la circonférence de la phalloplastie (1) dans le domaine de l'extrémité distale de la phalloplastie (1) dans le cas d'une phalloplastie (1) sans plastie du gland
ou
- au niveau de la circonférence de la phalloplastie (1) au voisinage d'une couronne de plastie du gland (12) de la phalloplastie (1);
où l'entretoise de soutien (25) est adaptée à la longueur de la phalloplastie (1) et formée pour s'étendre le long de l'axe longitudinal (10) de la phalloplastie (1) entre les éléments d'appui proximal et distal (21, 23); et où une première extrémité proximale (251) de la au moins une entretoise de soutien (25) est reliée à l'élément d'appui proximal (21) et une seconde extrémité distale (253) est reliée à l'élément d'appui distal (23);
**caractérisé en ce que**
l'élément d'appui proximal (21) est formé comme une section annulaire en forme d'arc de forme anatomique avec un collier de soutien (211) s'y raccordant suivant la circonférence pour le soutien proximal de la phalloplastie (1) et est adapté à la circonférence et à la forme de l'attache inférieure de la phalloplastie (1) à l'os pubien (11) de l'homme trans; et
que l'élément de maintien élastique (4) est formé pour être disposé dans le domaine de l'élément d'appui distal (23) et, conjointement avec celui-ci, former une structure annulaire fermée pour l'enveloppement circonférentiel et le soutien distal de la phalloplastie (1).

2. Dispositif pour rigidifier le pénis (3) d'un homme biologique, comprenant:
au moins un support de soutien rigide (2) et au moins deux éléments de maintien élastiques (4),
où le support de soutien (2) comprend:
- un élément d'appui proximal (21),
- un élément d'appui distal (23), et
- au moins une entretoise de soutien (25),
où l'élément d'appui distal (23) est formé comme une section annulaire en forme d'arc de forme anatomique pour le soutien distal, qui est formée au niveau de la circonférence du pénis (3) dans le domaine de la transition avec le gland (33);
où l'entretoise de soutien (25) est adaptée à la longueur du corps du pénis (35) et est formée pour s'étendre le long de l'axe longitudinal du corps du pénis (35) entre les éléments d'appui proximal et distal (21, 23),
où une première extrémité proximale (251) de la au moins une entretoise de soutien (25) est reliée à l'élément d'appui proximal (21) et une seconde extrémité distale (253) est reliée à l'élément d'appui distal (23); et
**caractérisé en ce que**
l'élément d'appui proximal (21) est formé comme une section annulaire en forme d'arc de forme anatomique pour le soutien proximal du pénis (3), qui est adaptée à la circonférence de l'attache inférieure du pénis (3) avec le testicule (34) de l'homme; et que les au moins deux éléments de maintien élastiques (4) sont chacun formés pour être disposés dans le domaine de l'élément d'appui distal et proximal (21, 23) et pour former avec celui-ci une structure annulaire fermée pour l'enveloppement circonférentiel et le soutien du pénis (3).

3. Dispositif selon la revendication 1 ou 2, où le support de soutien (2) est formé d'un matériau avec une rigidité en flexion supérieure à celle du matériau de l'élément de maintien élastique.

4. Dispositif selon l'une des revendications 1 à 3, où le support de soutien (2) est formé d'un matériau plastique approprié au contact avec la peau et, en particulier, comprend au moins un matériau choisi dans le groupe de: PETG, polyamide, PEEK et polypropylène.

5. Dispositif selon l'une des revendications précédentes, où l'élément de maintien élastique (4) comprend au moins un matériau choisi dans le groupe du silicone et du polyuréthane.

6. Dispositif selon l'une des revendications précédentes, où l'élément d'appui proximal (21) est formé comme une section annulaire en forme d'arc ouverte vers le haut avec un collier de soutien adjacent à celle-ci pour entourer en dessous le domaine d'attache proximal de la phalloplastie (1) et pour former un espace de réception (210) pour le domaine proximal de la phalloplastie (1) d'un homme trans.

7. Dispositif selon l'une des revendications précédentes, où l'élément d'appui distal (23) est formé comme une section annulaire en forme d'arc ouverte vers le bas pour entourer au dessus le domaine distal de la phalloplastie (1) d'un homme trans et pour former un espace de réception ouvert vers le bas (230).

8. Dispositif selon l'une des revendications précédentes, où le support de soutien (2) comprend au moins deux entretoises de soutien (25) qui s'étendent chacune d'une extrémité supérieure distale de l'élément d'appui proximal (21) à une extrémité inférieure proximale de l'élément d'appui distal (23), où, de préférence, les au moins deux entretoises de soutien (25) sont formées pour s'étendre le long de côtés opposés de la phalloplastie (1).

9. Dispositif selon l'une des revendications précédentes, où les deux éléments d'appui (21, 23) et la au moins une entretoise de soutien (2) sont formés d'une seule pièce en un matériau.

10. Dispositif selon l'une des revendications précédentes, où au niveau de l'élément d'appui distal (23) est formé au moins un évidement (232) et/ou au moins une saillie (233) pour la fixation de l'élément de maintien élastique (4) à l'élément d'appui distal (23).

11. Dispositif selon l'une des revendications précédentes, où le support de soutien (2) est produit au moyen d'un procédé d'impression tridimensionnelle ou au moyen d'un procédé de moulage par injection.

12. Dispositif selon l'une des revendications précédentes, où le support de soutien (2) est produit au moyen d'un procédé d'impression tridimensionnelle et où le au moins un élément élastique (4) est également produit au moyen d'un procédé d'impression tridimensionnelle et imprimé sur le support de soutien (2).

13. Procédé de fabrication d'un dispositif selon l'une des revendications 1 ou 3 à 12, comprenant les étapes:
a) mesure de la circonférence et de la forme de l'attache inférieure d'une phalloplastie (1) à l'os pubien (11) d'un homme trans;
b) mesure de la circonférence de la phalloplastie (1) de l'homme trans sans plastie du gland (13) dans le domaine de l'extrémité distale de la phalloplastie (1) ou mesure de la circonférence de la phalloplastie (1) de l'homme trans avec plastie du gland (13) au voisinage de la couronne de plastie du gland (12);
c) mesure de la circonférence de la phalloplastie (1) au milieu de la phalloplastie (19) entre l'attache proximale et l'extrémité distale de la phalloplastie (1);
d) mesure de la longueur de la phalloplastie (1) de l'attache à la pointe de la phalloplastie (1);
e) production du support de soutien (2) et, de préférence, de l'élément de maintien élastique (4) pour la phalloplastie (2) à l'aide des mesures des étapes a) à d).

14. Procédé de fabrication d'un dispositif selon l'une des revendications 2 à 12, comprenant les étapes:
a) mesure de la circonférence et de la forme de l'attache proximale inférieure d'un pénis avec l'os pubien d'un homme biologique;
b) mesure de la circonférence du pénis dans le domaine de la transition du corps du pénis au gland;
c) mesure de la circonférence du corps du pénis au milieu entre l'attache proximale et la transition avec le gland;
d) mesure de la longueur du corps du pénis de l'attache proximale à la transition avec le gland;
e) production du support de soutien et, de préférence, des éléments de maintien élastiques pour le pénis à l'aide des mesures des étapes a) à d).

15. Procédé selon la revendication 13 ou 14, où le procédé selon l'étape d) comprend en outre l'étape de procédé: e) création d'un modèle du support de soutien, où le dimensionnement des deux éléments d'appui, de la au moins une entretoise de soutien et, de préférence, de l'élément de maintien élastique a lieu sur la base des mesures des étapes de procédé a) à d); et, où à l'étape e), le support de soutien et, de préférence, l'élément de maintien élastique sont produits à l'aide du modèle.
